# EUROPÄISCHE PATENTSCHRIFT

(11) **EP 2 757 953 B1**
(45) Veröffentlichungstag und Bekanntmachung des Hinweises auf die Patenterteilung: **29.07.2015**
(21) Anmeldenummer: 12761617.5
(22) Anmeldetag: 19.09.2012
(51) Int. Cl.: A61B 5/15, A61B 5/151, B01L 3/00

(54) **VERFAHREN ZUM MASKENÄTZEN EINES STECHELEMENTS**
METHOD FOR THE MASK-ETCHING OF A PIERCING ELEMENT
PROCÉDÉ DE GRAVURE PAR MASQUE D'UN ORGANE DE PIQÛRE

(30) Priorität: 23.09.2011 EP 11182455
(43) Veröffentlichungstag der Anmeldung: 30.07.2014
(73) Patentinhaber: Roche Diagnostics GmbH, 68305 Mannheim (DE); F.Hoffmann-La Roche AG, 4070 Basel (CH)
(72) Erfinder: ZIPFEL, Marzellinus, 79112 Freiburg (DE); LOPEZ MRAS, Angel, 75180 Pforzheim (DE)
(74) Vertreter: Pfiz, Thomas
(86) Internationale Anmeldenummer: PCT/EP2012/068426
(87) Internationale Veröffentlichungsnummer: WO 2013/041557

(56) Entgegenhaltungen:
- EP-A1- 2 263 544
- EP-A1- 2 272 429
- WO-A1-2006/066744

## Beschreibung

Die Erfindung betrifft ein Verfahren zum Maskenätzen eines Stechelements, das einen langgestreckten Schaft, eine distal abstehende Spitze und einen längs des Schafts bis in den Bereich der Spitze verlaufenden, seitlich offenen Sammelkanal für Körperflüssigkeit aufweist, bei dem auf die beiden Seiten eines Substrats jeweils eine Seite einer doppelseitigen Ätzmaske aufgebracht wird und unter Einwirkung eines Ätzmittels das Stechelement als Formätzteil gebildet wird, wobei eine Kanalseite der Ätzmaske mit einem Kanalätzschlitz zum einseitigen Ätzen des Sammelkanals versehen wird. Die Erfindung betrifft weiter ein entsprechend hergestelltes Stechelement.

Ein solches Ätzverfahren wird in der WO2006/066744 A1 vorgeschlagen, um disposible Stechelement für die Gewinnung geringer Probenmengen bereitzustellen, wie sie beispielsweise für Blutglucosebestimmungen in situ als Kapillarblut aus einem Hauteinstich entnommen werden. Dort ist allerdings ein Ätzmaskenlayout für einen frontseitig offenen und proximal geschlossenen Kapillarkanal vorgesehen, wobei die Stechspitze auf der von dem Kanal abgewandten Rückseite distal vorspringt. Problematisch ist dabei auch die Vermeidung von Störkonturen beim Stechvorgang durch die vorderseitig aufragenden Kanalwände.

Die EP-A 2 272 429 beschreibt ein Stechelement in Form einer Lanzette mit einer Rinne als Kanal, der sich in einem Abschnitt im Bereich der Schneide kontinuierlich verjüngt. Ein Stechelement als geätztes Flachformteil mit proximal abstehenden Armen zur Ankopplung eines Stechantriebs ist aus der EP-A 2 263 544 bekannt.

Ausgehend hiervon liegt der Erfindung die Aufgabe zugrunde, die im Stand der Technik bekannten Ätzverfahren und damit erzeugten Stechelemente weiter zu optimieren und hinsichtlich der Probenaufnahme bzw. Probenübergabe in integrierten diagnostischen Systemen zu verbessern.

Zur Lösung dieser Aufgabe wird die in den unabhängigen Patentansprüchen angegebene Merkmalskombination vorgeschlagen. Vorteilhafte Ausgestaltungen und Weiterbildungen der Erfindung ergeben sich aus den abhängigen Ansprüchen.

Die Erfindung geht von dem Gedanken aus, den Sammelkanal für die Probenaufnahme und -übergabe endseitig zu optimieren. Dementsprechend wird erfindungsgemäß vorgeschlagen, dass ein proximaler und/oder distaler Endabschnitt des Kanalätzschlitzes zum Schlitzende hin verjüngt ausgebildet wird, dass die Ätzmaske mit einem in proximaler Richtung an den Kanalätzschlitz anschließenden Flanschbildungsbereich versehen wird, und dass durch Unterätzen des Flanschbildungsbereichs eine die Mündungsstelle des Sammelkanals bildende Flanschkante an einem proximalen Halteteil des Stechelements erzeugt wird.

Dadurch kann einerseits gewährleistet werden, dass während des Ätzverfahrens im vorderen distalen Spitzenbereich der Kanalquerschnitt sich verjüngt oder zumindest gleichbleibt und keine knochenförmige Erweiterung aufgrund von Strömungsprozessen des Ätzmittels am Kanalende auftritt. Auch wenn eine solche "Knochenbildung" für die Flüssigkeitsaufnahme im Spitzenbereich zunächst als unkritisch einzustufen wäre, könnte dies jedoch im Bereich der Spitze aufgrund der reduzierten Materialstärke zum Durchätzen führen. Dementsprechend könnte der Sammelkanal nicht weit nach vorne geführt werden, und der vergrößerte Abstand zwischen Spitze und Kanalende müsste zu einer tieferen Einstechtiefe und damit erhöhtem Schmerzempfinden führen. Um die Einstechtiefe zu reduzieren und eine optimale Proben- bzw. Blutaufnahme zu gewährleisten, ist es somit entscheidend, dass der Kapillarkanal bereits während des Einstichs unter der Haut mit Blut bzw. Gewebeflüssigkeit in Kontakt kommt. Eine solche Ätzteilgestaltung wird durch eine Ätzmaske erreicht, deren Kanalätzschlitz bis in den distalen Spitzenbereich geführt wird und sich dabei verjüngt.

Andererseits wird durch die Ätzschlitzverjüngung am proximalen Ende erreicht, dass der geätzte Sammelkanal am hinteren Ende mit gleichbleibendem oder sogar abnehmendem Kapillardurchmesser endet, so dass der Kapillartransport nicht vorzeitig unterbrochen wird, wie dies bei einer Kapillarerweiterung durch übliche Ätzverfahren unvermeidbar wäre.

Eine vorteilhafte Ausgestaltung sieht vor, dass der Endabschnitt des Kanalätzschlitzes linear verjüngt wird, so dass der geätzte Sammelkanal in Richtung der Verjüngung mit gleichbleibender oder kontinuierlich abnehmender Querschnittsfläche verläuft.

Um einen kapillaraktiven Probenübertrag zu unterstützen, ist es von Vorteil, wenn der Kanalätzschlitz in einer proximale Partie der Ätzmaske so positioniert wird, dass der geätzte Sammelkanal stirnseitig offen an dem Halteteil mündet.

Eine weitere Verbesserung hinsichtlich der Probenhandlings in einer integrierten Testkonfiguration lässt sich dadurch erreichen, dass eine die Mündungsstelle des Sammelkanals bildende, gerade Flanschkante insbesondere zum Anflanschen eines Testelements an dem Halteteil erzeugt wird.

Um die Mündungsstelle planar zu begrenzen, ist es vorteilhaft, wenn der Flanschbildungsbereich eine quer zu dem Kanalätzschlitz über dessen proximales Schlitzende verlaufende Maskenbrücke aufweist.

Eine weitere Verbesserung hinsichtlich einer definierten Gestaltung des Halteteils sieht vor, dass die Ätzmaske mit einem dem Kanalätzschlitz im proximalen Abstand nachgeordneten Opferfortsatz zur Abschirmung einer Ätzmitteleinwirkung im Mündungsbereich des Sammelkanals versehen wird.

Zur radialen Abschirmung eines Halbraums hinter einer Maskenkante ist es vorteilhaft, wenn der an der Maskenkante frei abstehende Opferfortsatz in einem von dem proximalen Schlitzende des Kanalätzschlitzes abgewandten Randbereich bogenförmig, insbesondere kreisbogenförmig begrenzt wird. Dabei sollte die Dimensionen des Opferfortsatzes so an die Unterätzweite des Ätzmittels angepasst werden, dass der Opferfortsatz bis zu seiner proximalen Basis vollständig abgeätzt wird. Grundsätzlich sind auch andere Geo-metrien des Opferfortsatzes denkbar, beispielsweise rechteckig oder dreieckig, um eine abschirmende Fläche bereitzustellen, die einen geeigneten Kantenabstand und eine gewisse Ausdehnung besitzt, um den Mündungsbereich des Sammelkanals vor dem Ätzmedium zu schützen.

Eine weitere bevorzugte Ausführung sieht vor, dass die Ätzmaske auf beiden Substratseiten mit einem Spitzenbildungsbereich zur Ausbildung einer Spitzenkontur versehen wird, wobei der kanalseitige Spitzenbildungsbereich dem auf der Gegenseite liegenden Spitzenbildungsbereich distal vorgelagert ist. Auf diese Weise steht genügend Substratmaterial auch im Bereich der Spitze für einen möglichst weit vorgezogenen Sammelkanal zur Verfügung, während die Schneidwirkung nicht durch Störkonturen auf der Gegenseite behindert wird.

Um etwaige Störkonturen weiter zu reduzieren, ist es von Vorteil, wenn die Ätzmaske auf ihrer von dem Kanalätzschlitz abgewandten Gegenseite eine Hilfsöffnung insbesondere zur Vermeidung von hinterschnittenen Kanten im Bereich der Spitze aufweist. Vorteilhafterweise wird die Hilfsöffnung in einem Spitzenbildungsbereich der Ätzmaske im lateralen Abstand zu einer Maskenkante angeordnet. In diesem Zusammenhang ist es besonders günstig, wenn die Hilfsöffnung zwei in distaler Richtung V-förmig aufeinander zulaufende Hilfsöffnungsschenkel und einen in distaler Richtung über die Hilfsöffnungsschenkel hinaus sich erstreckenden, vorzugsweise als Schlitz oder Lochreihe ausgebildeten distalen Hilfsöffnungsfortsatz aufweist.

Zur Vermeidung von Widerhaken bzw. unerwünschten Höckern sollte das Ende des Hilfsöffnungsfortsatzes in proximaler Richtung gesehen hinter dem distalen Ende der zu bildenden Spitze liegen.

Demzufolge ist es günstig, wenn die Hilfsöffnung Y-förmig ausgebildet wird, wobei der Verbindungspunkt der aufeinander zulaufenden Öffnungsschenkel in einem distalen Abstand von der zu bildenden Spitzenkontur angeordnet wird.

Für die Probenaufnahme bereits in der Haut ist es besonders vorteilhaft, wenn der Kanalätzschlitz so weit in einen distalen Spitzenbildungsbereich der Ätzmaske erstreckt wird, dass der Sammelkanal in einem Abstand von 50 bis 1000 µm, bevorzugt 150 bis 400 µm vor dem distalen Ende der Spitze endet.

Eine weitere vorteilhafte Ausgestaltung sieht vor, dass der verjüngte Endabschnitt des Kanalätzschlitzes über eine Länge im Bereich zwischen 100 und 300 µm um das 0,4- bis 0,6-fache seiner Anfangsbreite zu einer stumpfen Endkante hin in der Breite reduziert wird.

Ein weiterer Erfindungsaspekt betrifft ein Stechelement mit einem langgestreckten Schaft, einer distal abstehenden Spitze, einem proximalen Halteteil und einem längs des Schafts bis in den Bereich der Spitze verlaufenden, seitlich offenen Sammelkanal für Körperflüssigkeit, wobei der Sammelkanal an mindestens einem Endabschnitt mit kontinuierlich abnehmender Querschnittfläche verläuft und durch ein Maskenätzverfahren insbesondere nach einem der vorhergehenden Ansprüche gebildet ist.

Im Folgenden wird die Erfindung anhand des in der Zeichnung schematisch dargestellten Ausführungsbeispiels näher erläutert. Es zeigen
- Fig. 1: einen distalen kanalseitigen Maskenabschnitt einer Ätzmaske zur Herstellung eines mit einem Sammelkanal versehenen Stechelements in der Draufsicht;
- Fig. 2: einen mit dem Maskenabschnitt nach Fig. 1 erzeugten Bereich des Stechelements;
- Fig. 3: einen distalen rückseitigen Maskenabschnitt in einer Fig. 1 entsprechenden Darstellung;
- Fig. 4: einen mit dem Maskenabschnitt nach Fig. 3 erzeugten Bereich des Stechelements;
- Fig. 5: einen proximalen kanalseitigen Maskenabschnitt in der Draufsicht;
- Fig. 6: einen mit dem Maskenabschnitt nach Fig. 5 erzeugten Bereich des Stechelements;
- Fig. 7 und 8: einen Querschnitt durch die Ätzmaske und das Stechelement entlang der Schnittlinien 7 - 7 und 8 - 8 von Fig. 9;
- Fig. 9: das als Formätzteil gebildete Stechelement in Verbindung mit einem proximal angeflanschten Testelement in perspektivischer Darstellung.

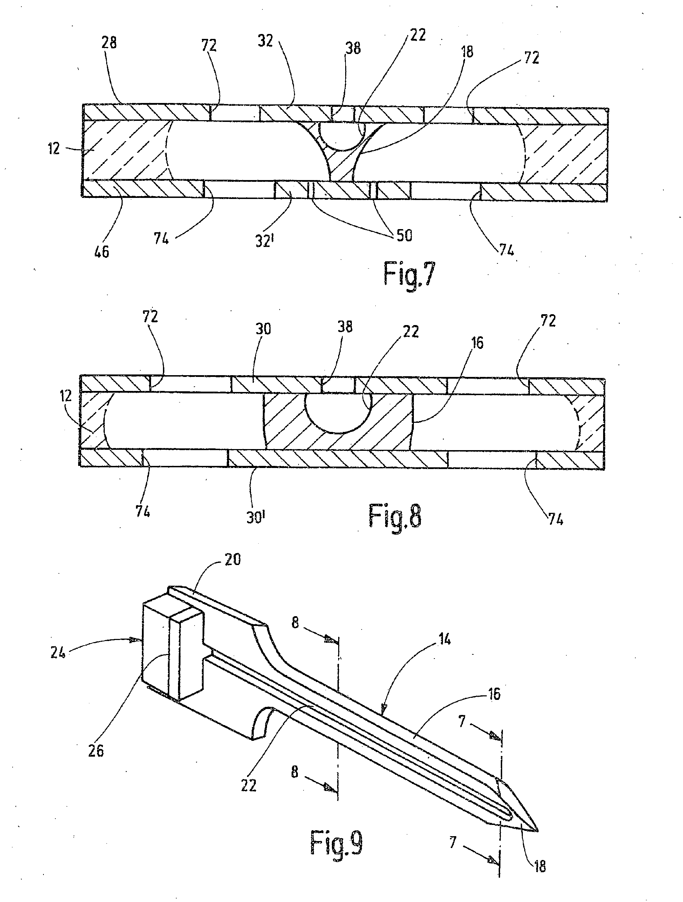

Die in der Zeichnung dargestellte Ätzmaske 10 wird als doppelseitiges Layout auf die beiden Seiten eines dünnen Edelstahl-Substrats 12 aufgebracht, um Maskenätzen ein Stechelement 14 als Formätzteil zu bilden, das gemäß Fig. 9 einen langgestreckten Schaft 16, eine distal abstehende Spitze 18, einen proximalen Halteteil 20 und einen längs des Schafts 16 bis in den Bereich der Spitze 18 verlaufenden, über seien Länge halboffenen bzw. rillenförmigen Sammelkanal 22 zum Sammeln von Körperflüssigkeit (Blut, Gewebeflüssigkeit) bei einem Hauteinstich aufweist. Das Stechelement 14 lässt sich als integriertes diagnostisches Verbrauchsmittel mit einem Testelement 24 kombinieren, um die in dem kapillaren Sammelkanal 22 aufgenommene Probenflüssigkeit auf eine Reagenzschicht 26 zur Bestimmung eines Analyten (z.B. Glucose) zu übertragen. Dabei kann das Testelement 24 bereits anfänglich fest an dem Stechelement 14 integriert sein oder aber erst nachträglich zum Probentransfer mit dem Stechelement 14 in Verbindung gebracht werden.

Die Ätzmaske 10 kann durch Fotolithografie, d.h. durch Belichten und Auswaschen aus Fotolack in an sich bekannter Weise auf dem Substrat 12 strukturiert werden. Über die Freisparungen in der so erzeugten Ätzmaske 36 erfolgt anschließend eine Beaufschlagung des Substrats 12 mit einem Ätzmittel, wobei die abgedeckten bzw. maskierten Bereiche der Grundform nach freigeätzt werden. Hierbei ist zu berücksichtigen, dass der Materialabtrag nicht nur in die Tiefe, sondern auch durch Hinter- bzw. Unterätzen von Randkonturen der Ätzmaske 10 erfolgt. Durch äußere Einflussparameter bzw. Materialeigenschaften des Substrats kann der Ätzvorgang auch anisotrop erfolgen, d. h. die laterale Unterätzrate bzw. -weite ist dann größer oder kleiner als die Tiefenätzrate.

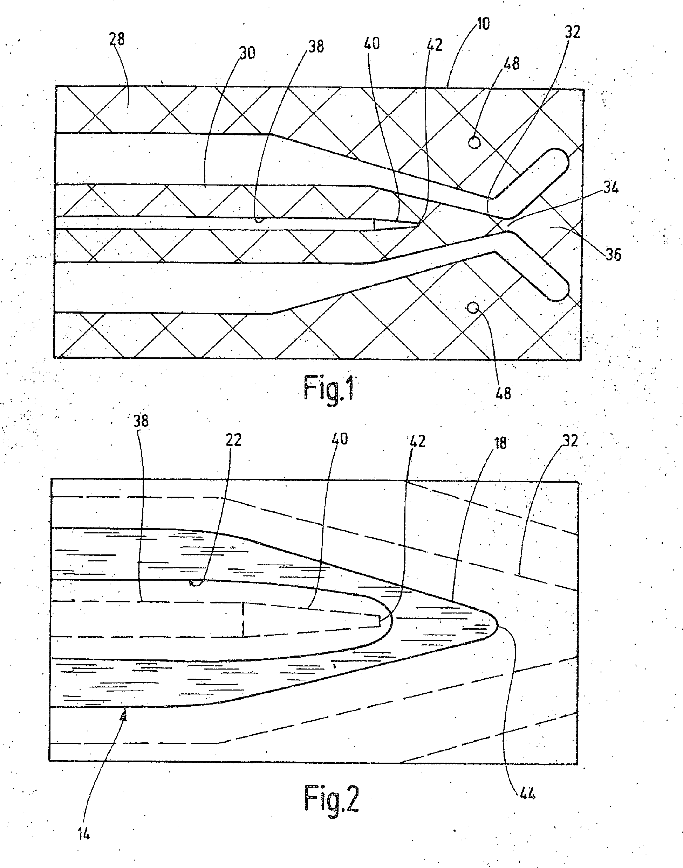

Die in Fig. 1 in einem distalen Ausschnitt gezeigte Kanalseite 28 der Ätzmaske 10 weist einen Schaftbildungsbereich 30 für den Schaft 16 und einen Spitzenbildungsbereich 32 zur Bildung der Spitze 18 auf. Ausgehend von einer Engstelle 34 folgt auf den Spitzenbildungsbereich 32 in distaler Richtung ein sich erweiternder Abschirmbereich 36, der ein frontales Abätzen der Spitze 18 verhindert, so dass eine besonders scharfe Spitzenkontur nur durch seitliches Unterätzen des Spitzenbildungsbereichs 32 freigeätzt wird.

Weitere Einzelheiten einer solchen Abschirmung zur Spitzenkonturierung gehen auch aus der WO2006/066744 hervor, auf die in diesem Zusammenhang Bezug genommen wird. Dort ist allerdings der distal vorspringende Spitzenbereich auf der von dem Kapillarkanal abgewandten (dort als Rückseite bezeichneten) Substratseite angeordnet, während der frontal offene Kapillarkanal erst hinter der Spitze seitlich begrenzt ist.

In dem erfindungsgemäßen Maskenlayout ist auf der Kanalseite 28 ein Kanalätzschlitz 38 zum einseitigen (rillenförmigen) Ätzen eines im Bereich der Spitze 18 endenden, stirnseitig geschlossenen Sammelkanals 22 vorgesehen. Zu diesem Zweck ist ein distaler Endabschnitt 40 des Kanalätzschlitzes 38 zu dem im Spitzenformungsbereich 32 liegenden distalen Schlitzende 42 hin verjüngt ausgebildet.

Fig. 2 zeigt einen vergrößerten Ausschnitt der Ätzmaske 10 nach Fig. 1 in gestrichelten Linien in Verbindung mit dem freigeätzten distalen Abschnitt des Stechelements 14. Daraus ist ersichtlich, dass die lineare Verjüngung des Endabschnitts 40 des Kanalätzschlitzes 38 so gewählt ist, dass der geätzte Sammelkanal 22 mit kontinuierlich abnehmender Breite und Tiefe bis in den Bereich der Spitze 18 verläuft.

Zweckmäßig liegt die Substratdicke im Bereich von 100 bis 300 µm. Die Breite des Kanalätzschlitzes 38 im Mittelteil kann etwa 100 bis 150 µm betragen, während der verjüngte Endabschnitt 40 über eine etwa der Substratdicke entsprechende Länge von 100 bis 300 µm um etwa die Hälfte seiner Anfangsbreite zu einer das Schlitzende 42 bildenden stumpfen Endkante hin in seiner Breite reduziert wird. Dabei kann erreicht werden, dass der Sammelkanal 22 in einem geringen Abstand im Bereich von 150 bis 400 µm vor dem distalen Ende 44 der Spitze 18 endet.

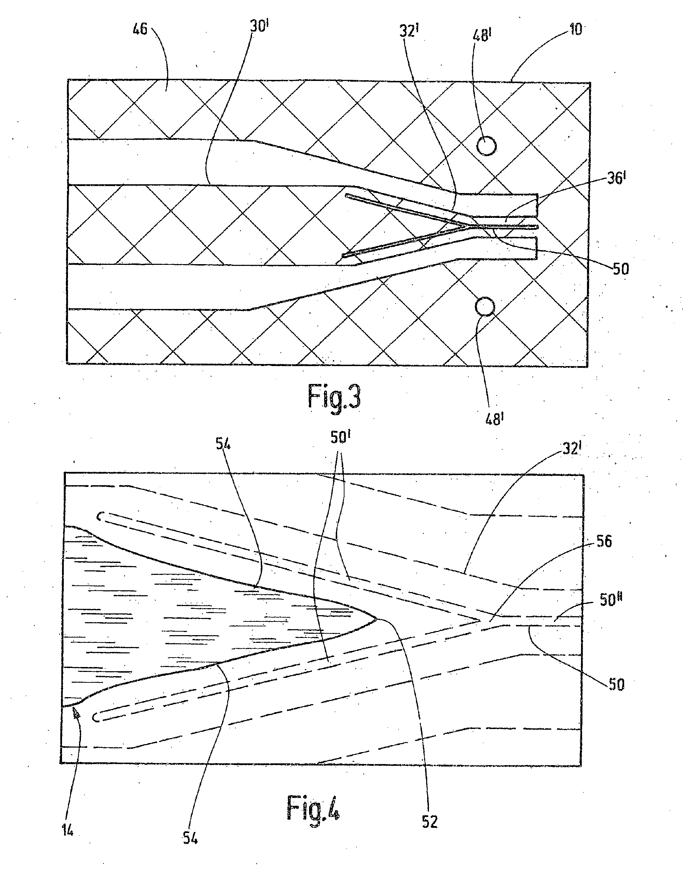

Fig. 3 zeigt einen distalen Abschnitt der Ätzmaske 10 auf der von der Kanalseite 28 abgewandten Gegenseite 46. Die beiden Maskenseiten 28, 46 sind dabei so zueinander positioniert, dass die Positionier- bzw. Indexlöcher 48, 48' konzentrisch ineinander liegen. Auch auf der Gegenseite 46 weist die Ätzmaske 10 einen Schaftbildungsbereich 30' für den Schaft 16 und einen Spitzenbildungsbereich 32' für die Bildung einer Spitzenkontur auf. Dem Spitzenbildungsbereich 32' ist ein Abschirmbereich 36' distal vorgelagert, um eine frontale Abstumpfung zu vermeiden. Zusätzlich ist eine Y-förmig ausgebildete Hilfsöffnung 50 zur Vermeidung von hinterschnittenen Kanten und Höckern im Bereich der Spitzenkontur auf der Kanalgegenseite 46 vorgesehen, wie es nachstehend näher erläutert wird.

Der Spitzenbildungsbereich 32' auf der Gegenseite 46 ist gegenüber dem kanalseitigen Spitzenbildungsbereich 32 in proximaler Richtung zurückversetzt, so dass die Spitze 18 in Längsrichtung gesehen ausgehend von dem distalen Ende 44 eine konvexe Abrundung erhält.

Wie aus Fig. 4 ersichtlich, wird durch den Spitzenbildungsbereich 32' auf der Gegenseite 46 an dem Stechelement 14 eine zu einem Scheitelpunkt 52 über Kanten 54 keilförmig zulaufende Spitzenkontur erzeugt. Der Scheitelpunkt 52 liegt dabei zweckmäßig um mehr als die Substratdicke in proximaler Richtung hinter dem kanalseitigen distalen Ende 44. Die etwa parallel zu den Kanten 54 aufspreizenden Öffnungsschenkel der Hilfsöffnung 50 sorgen dafür, dass durch zusätzlich eindringendes Ätzmittel die Kanten 54 im Querschnitt konvex abgerundet werden und keine Hohlkehle ausbilden. Entsprechend ist der Verbindungspunkt 56 der aufeinander zulaufenden Öffnungsschenkel 50' dem Scheitelpunkt 52 distal vorgelagert, so dass auch in Längsrichtung kein den Einstich behindernder hinterschnittener Widerhaken entsteht. Würde der Verbindungspunkt 56 weiter vorne angeordnet, wäre kein solcher Effekt mehr zu erzielen. Hingegen entstünde bei einer Verlagerung weiter nach hinten eine Art von Querkanal mit einem davorliegenden Höcker. Um eine solche Störkontur für den Nadeleinstich zu vermeiden, besitzt die Hilfsöffnung 50 ausgehend von dem Verbindungspunkt 56 einen distalen Basisschenkel bzw. Hilfsöffnungsschlitz 50", der sich vorzugsweise mit gleichförmiger Breite in distaler Richtung erstreckt. Gegenüber dem kanalseitigen distalen Ende 44 der zu bildenden Spitze 18 endet der Hilfsöffnungsschlitz 50" in einem proximalen Abstand. Damit wird der größeren Breite des kanalseitigen Spitzenbildungsbereichs 32 Rechnung getragen.

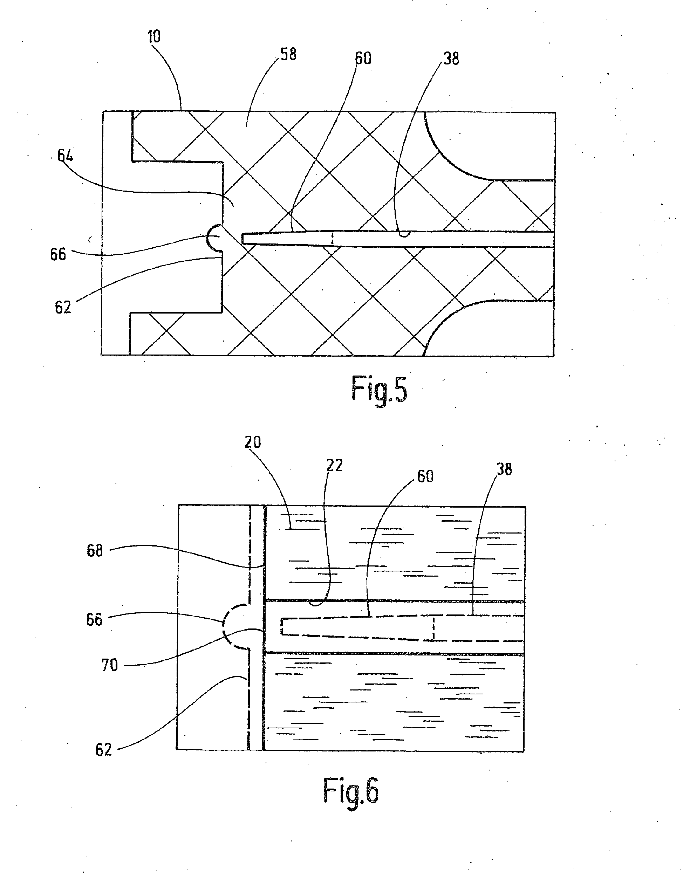

Fig. 5 zeigt eine proximale (hintere) Partie 58 der Ätzmaske 10 auf der Kanalseite 28, die zur Erzeugung des Halteteils 20 vorgesehen ist. Der Kanalätzschlitz 38 läuft dort in einem in proximaler Richtung verjüngten Endabschnitt 60 aus. Grundsätzlich kann dieser hintere Endabschnitt 60 entsprechend dem vorderen, distalen Endabschnitt 40 dimensioniert werden. Dabei kann der Kanalätzschlitz 38 derart in der Maskenpartie 58 positioniert werden, dass der geätzte Sammelkanal 22 stirnseitig offen an dem Halteteil 20 mündet, um einen linearen Kapillartransport der Probenflüssigkeit zu dem Testelement 24 hin zu ermöglichen.

Zum Andocken des Testelements 24 sollte der Mündungsbereich des Sammelkanals 22 möglichst keine Querschnittserweiterung aufweisen, die den kapillaren Flüssigkeitstransport stoppen könnte. Ebenso sollte das Testelement 24 flächig ohne Luftspalte anliegen. Um dies zu erreichen, ist die Ätzmaske 10 mit einem in proximaler Richtung an den Kanalätzschlitz 38 anschließenden Flanschbildungsbereich 62 versehen. Der Flanschbildungsbereich 62 weist eine quer zu dem Kanalätzschlitz 38 über dessen proximales Schlitzende verlaufende Maskenbrücke 64 auf, die bei gleichmäßiger Unterätzung in distaler Richtung zu einer geradlinigen Randkontur führt.

Zusätzlich zu der distalen Unterätzung strömt jedoch das Ätzmittel über den Kanalätzschlitz 38 auch in proximaler Richtung und würde an der Mündungsstelle zu einer entsprechenden Ätzverbreiterung führen. Um diesen Effekt zu kompensieren, ist die Ätzmaske 10 mit einem dem Kanalätzschlitz 38 im proximalen Abstand nachgeordneten Opferfortsatz 66 versehen, der eine distale Ätzmitteleinwirkung im Mündungsbereich des Sammelkanals 22 verhindert.

Um dies zu erreichen, ist der an der Maskenbrücke 64 frei abstehende Opferfortsatz 66 in einem proximalen Randbereich bogenförmig ausgebildet, wobei der Radius an die Unterätzweite angepasst ist, so dass der Opferfortsatz 66 vollständig abgeätzt wird.

Wie in Fig. 6 veranschaulicht, wird durch das vorstehend beschriebene Maskenlayout eine gerade Flanschkante 68 in dem Halteteil 20 erzeugt, die zu einem optimierten Probenübertrag auf das angeflanschte Testelement 24 führt. Dabei sorgt der Opferfortsatz 66 nicht nur für eine geradlinige Begrenzung der Mündungsstelle 70 des Sammelkanals 22, sondern verhindert auch eine zusätzliche Ätzverbreiterung des Kanalendabschnitts. Es versteht sich, dass die für die Gegenseite nicht gezeigte proximale Partie der Ätzmaske 10 keinen Kanalätzschlitz und demgemäß keinen Opferfortsatz aufweist, im Übrigen aber mit der kanalseitigen proximalen Partie 58 übereinstimmt.

Wie in Fig. 7 und 8 weiter verdeutlicht, werden die Bauteilumrisse und Topographie des Stechelements 14 durch das beiderseitig unterschiedliche Maskenlayout auf dem Substrat 12 bestimmt. Neben der einseitigen Oberflächenätzung des feinen Sammelkanals 22 wird durch die Tiefenätzung über die Maskenöffnungen 70, 72 auch eine Separation des Formätzteils in dem Substrat erreicht, wobei durch eine entsprechende Maskenwiederholung auch große Stückzahlen gegebenenfalls von Rolle-zu-Rolle gefertigt werden können.

## Patentansprüche

1. Verfahren zum Maskenätzen eines Stechelements (14), das einen langgestreckten Schaft (16), eine distal abstehende Spitze (18) und einen längs des Schafts (16) bis in den Bereich der Spitze (18) verlaufenden, seitlich offenen Sammelkanal (22) für Körperflüssigkeit aufweist, bei dem auf die beiden Seiten eines Substrats (12) eine doppelseitige Ätzmaske (10) aufgebracht wird und unter Einwirkung eines Ätzmittels das Stechelement (14) als Formätzteil gebildet wird, wobei eine Kanalseite (28) der Ätzmaske (10) mit einem Kanalätzschlitz (38) zum einseitigen Ätzen des Sammelkanals (22) versehen wird, **dadurch gekennzeichnet, dass** ein proximaler und/oder distaler Endabschnitt (40,60) des Kanalätzschlitzes (38) zum Schlitzende hin verjüngt ausgebildet wird, dass die Ätzmaske (10) mit einem in proximaler Richtung an den Kanalätzschlitz (38) anschließenden Flanschbildungsbereich (62) versehen wird, und dass durch Unterätzen des Flanschbildungsbereichs (62) eine die Mündungsstelle des Sammelkanals (22) bildende Flanschkante (68) an einem proximalen Halteteil (20) des Stechelements (14) erzeugt wird.

2. Verfahren nach Anspruch 1, **dadurch gekennzeichnet, dass** der Endabschnitt (40,60) des Kanalätzschlitzes (38) linear verjüngt wird, so dass der geätzte Sammelkanal (22) in Richtung der Verjüngung mit gleichbleibender oder kontinuierlich abnehmender Querschnittsfläche verläuft.

3. Verfahren nach Anspruch 1 oder 2, **dadurch gekennzeichnet, dass** der Kanalätzschlitz (38) in einer proximale Partie (58) der Ätzmaske (10) so positioniert wird, dass der geätzte Sammelkanal (22) stirnseitig offen an dem Halteteil (20) mündet.

4. Verfahren nach einem der Ansprüche 1 bis 3, **dadurch gekennzeichnet, dass** eine die Mündungsstelle des Sammelkanals (22) bildende gerade Flanschkante (68) insbesondere zum Anflanschen eines Testelements (24) an dem Halteteil (20) erzeugt wird.

5. Verfahren nach einem der Ansprüche 1 bis 4, **dadurch gekennzeichnet, dass** der Flanschbildungsbereich (62) eine quer zu dem Kanalätzschlitz (38) über dessen proximales Schlitzende verlaufende Maskenbrücke aufweist.

6. Verfahren nach einem der Ansprüche 1 bis 5, **dadurch gekennzeichnet, dass** die Ätzmaske (10) mit einem dem Kanalätzschlitz (38) im proximalen Abstand nachgeordneten Opferfortsatz (66) zur Abschirmung einer Ätzmitteleinwirkung im Mündungsbereich des Sammelkanals (22) versehen wird.

7. Verfahren nach Anspruch 6, **dadurch gekennzeichnet, dass** der an einer Maskenkante frei abstehende Opferfortsatz (66) in einem von dem proximalen Schlitzende (60) des Kanalätzschlitzes (38) abgewandten Randbereich bogenförmig, insbesondere kreisbogenförmig begrenzt wird.

8. Verfahren nach Anspruch 6 oder 7, **dadurch gekennzeichnet, dass** die Dimensionen des Opferfortsatzes (66) so an die Unterätzweite des Ätzmittels angepasst werden, dass der Opferfortsatz (66) bis zu seiner proximalen Basis vollständig abgeätzt wird.

9. Verfahren nach einem der Ansprüche 1 bis 8, **dadurch gekennzeichnet, dass** die Ätzmaske (10) auf ihrer von dem Kanalätzschlitz (38) abgewandten Gegenseite (46) eine Hilfsöffnung (50) insbesondere zur Vermeidung von hinterschnittenen Kanten im Bereich der Spitze (18) aufweist.

10. Verfahren nach Anspruch 9, **dadurch gekennzeichnet, dass** die Hilfsöffnung (50) in einem Spitzenbildungsbereich (32') der Ätzmaske (10) im lateralen Abstand zu einer Maskenkante angeordnet wird.

11. Verfahren nach Anspruch 9 oder 10, **dadurch gekennzeichnet, dass** die Hilfsöffnung (50) zwei in distaler Richtung V-förmig aufeinander zulaufende Hilfsöffnungsschenkel (50') aufweist.

12. Verfahren nach einem der Ansprüche 9 bis 11, **dadurch gekennzeichnet, dass** die Hilfsöffnung (50) einen in distaler Richtung sich erstreckenden, vorzugsweise als Schlitz oder Lochreihe ausgebildeten distalen Hilfsöffnungsfortsatz (50") aufweist.

13. Verfahren nach Anspruch 12, **dadurch gekennzeichnet, dass** das proximale Ende des Hilfsöffnungsfortsatzes (50") in proximaler Richtung gesehen hinter dem distalen Ende (44) der zu bildenden Spitze (18) angeordnet wird.

14. Verfahren nach einem der Ansprüche 9 bis 13, **dadurch gekennzeichnet, dass** die Hilfsöffnung (50) Y-förmig ausgebildet wird, wobei der Verbindungspunkt (56) der aufeinander zulaufenden Öffnungsschenkel in einem distalen Abstand von der zu bildenden Spitzenkontur (52) der Kanalgegenseite (46) angeordnet wird.

15. Stechelement (14) mit einem langgestreckten Schaft (16), einer distal abstehenden Spitze (18), und einem längs des Schafts (16) bis in den Bereich der Spitze (18) verlaufenden, seitlich offenen Sammelkanal (22) für Körperflüssigkeit, wobei der Sammelkanal (22) an mindestens einem Endabschnitt (40,60) mit kontinuierlich abnehmender Querschnittsfläche verläuft, **dadurch gekennzeichnet, dass** ein proximaler Halteteil (20) mit einer die Mündungsstelle des Sammelkanals (22) bildenden Flanschkante (68) durch ein Maskenätzverfahren nach einem der vorhergehenden Ansprüche gebildet ist.

## Claims

1. A method for the mask-etching of a piercing element (14) which has an elongate shaft (16), a distally protruding tip (18) and a laterally open collecting channel (22) that collects bodily fluid and extends along the shaft (16) as far as the area of the tip (18), in which method a double-sided etching mask (10) is applied to the two sides of a substrate (12) and, under the action of an etching agent, the piercing element (14) is formed as a part made by chemical blanking, wherein a channel side (28) of the etching mask (10) is provided with a channel etching slit (38) for unilateral etching of the collecting channel (22), **characterized in that** a proximal and/or distal end portion (40, 60) of the channel etching slit (38) is designed tapering toward the end of the slit, that the etching mask (10) is provided with a flange-forming area (62), which adjoins the channel etching slit (38) in the proximal direction, and **in that**, by undercutting the flange-forming area (62), a flange edge (68) forming the mouth of the collecting channel (22) is generated onto a proximal holding part (20) of the piercing element (14).

2. The method as claimed in claim 1, **characterized in that** the end portion (40, 60) of the channel etching slit (38) tapers linearly, such that the etched collecting channel (22) extends in the direction of the taper with a constant or continuously decreasing cross-sectional area.

3. The method as claimed in claim 1 or 2, **characterized in that** the channel etching slit (38), in a proximal region (58) of the etching mask (10), is positioned such that the etched collecting channel (22) opens out at the front end on the holding part (20).

4. The method as claimed in one of claims 1 to 3, **characterized in that** a straight flange edge (68) forming the mouth of the collecting channel (22) is generated, in particular for flanging a test element (24) onto the holding part (20).

5. The method as claimed in one of the claims 1 to 4, **characterized in that** the flange-forming area (62) has a mask bridge extending, transversely with respect to the channel etching slit (38), across the proximal slit end thereof.

6. The method as claimed in one of claims 1 to 5, **characterized in that** the etching mask (10) is provided with a sacrificial continuation (66), which is arranged at a proximal distance downstream of the channel etching slit (38) and which serves to screen an etching agent effect in the mouth area of the collecting channel (22).

7. The method as claimed in claim 6, **characterized in that** the sacrificial continuation (66) protruding freely on a mask edge is defined by an arc shape, in particular a circular arc shape, in an edge area directed away from the proximal slit end (60) of the channel etching slit (38).

8. The method as claimed in claim 6 or 7, **characterized in that** the dimensions of the sacrificial continuation (66) are adapted to the undercutting width of the etching agent, such that the sacrificial continuation (66) is completely etched off to its proximal base.

9. The method as claimed in one of claims 1 to 8, **characterized in that** the etching mask (10), on its opposite side (46) directed away from the channel etching slit (38), has an auxiliary opening (50), in particular for avoiding undercut edges in the area of the tip (18).

10. The method as claimed in claim 9, **characterized in that** the auxiliary opening (50) is arranged in a tip-forming area (32') of the etching mask (10) at a lateral distance from a mask edge.

11. The method as claimed in claim 9 or 10, **characterized in that** the auxiliary opening (50) has two auxiliary opening limbs (50') extending toward each other in a V shape in the distal direction.

12. The method as claimed in one of claims 9 to 11, **characterized in that** the auxiliary opening (50) has a distal auxiliary opening continuation (50"), which extends in the distal direction and which is preferably designed as a slit or series of holes.

13. The method as claimed in claim 12, **characterized in that** the proximal end of the auxiliary opening continuation (50"), seen in the proximal direction, is arranged behind the distal end (44) of the tip (18) to be formed.

14. The method as claimed in one of claims 9 to 13, **characterized in that** the auxiliary opening (50) is Y-shaped, wherein the connection point (56) of the opening limbs extending toward each other is arranged at a distal distance from the tip contour (52) to be formed on the channel opposite side (46).

15. A piercing element (14) with an elongate shaft (16), a distally protruding tip (18) and a laterally open collecting channel (22) that collects bodily fluid and extends along the shaft (16) as far as the area of the tip (18), wherein the collecting channel (22), at least at one end portion (40, 60), extends with a continuously decreasing cross-sectional area, **characterized in that** a proximal holding part (20) with a flange edge (68) forming the mouth of the collecting channel (22) is formed by a mask-etching method as claimed in one of the preceding claims.

## Revendications

1. Procédé de gravure par masque d'un organe de piqûre (14), qui présente une tige allongée (16), une pointe d'extrémité distale (18) et un canal de collecte (22) ouvert latéralement pour un liquide corporel qui s'étend le long de la tige (16) jusque dans la région de la pointe (18), dans lequel un masque de gravure bilatéral (10) est appliqué sur les deux faces d'un substrat (12) et l'organe de piqûre (14) est formé comme pièce de gravure de forme sous l'action d'un agent de gravure, dans lequel un côté de canal (28) du masque de gravure (10) est muni d'une fente de gravure de canal (38) pour la gravure unilatérale du canal de collecte (22), **caractérisé en ce qu'**une partie d'extrémité proximale et/ou distale (40, 60) de la fente de gravure de canal (38) est réalisée avec un rétrécissement en direction de l'extrémité de la fente, **en ce que** le masque de gravure (10) est muni d'une région de formation de bride (62) se raccordant à la fente de gravure de canal (38) en direction proximale, et **en ce qu'**une arête de bride (68) formant le lieu d'embouchure du canal de collecte (22) est produite sur une partie de maintien proximale (20) de l'organe de piqûre (14) par sous-gravure de la région de formation de bride (62).

2. Procédé selon la revendication 1, **caractérisé en ce que** la partie d'extrémité (40, 60) de la fente de gravure de canal (38) est rétrécie linéairement, de telle manière que le canal de collecte gravé (22) s'étende en direction du rétrécissement avec une section transversale constante ou décroissante de façon continue.

3. Procédé selon la revendication 1 ou 2, **caractérisé en ce que** la fente de gravure de canal (38) est positionnée dans une partie proximale (58) du masque de gravure (10), de telle manière que le canal de collecte gravé (22) débouche de façon ouverte côté frontal à la partie de maintien (20).

4. Procédé selon l'une quelconque des revendications 1 à 3, **caractérisé en ce que** l'on produit une arête de bride droite (68) formant le lieu d'embouchure du canal de collecte (22) en particulier en vue d'assembler un élément de test (24) à la partie de maintien (20).

5. Procédé selon l'une quelconque des revendications 1 à 4, **caractérisé en ce que** la région de formation de bride (62) présente un pont de masque s'étendant transversalement à la fente de gravure de canal (38) au-dessus de son extrémité de fente proximale.

6. Procédé selon l'une quelconque des revendications 1 à 5, **caractérisé en ce que** le masque de gravure (10) est muni d'un prolongement sacrificiel (66) succédant à la fente de gravure de canal (38) à distance proximale pour la protection d'une action d'agent de gravure dans la région d'embouchure du canal de collecte (22).

7. Procédé selon la revendication 6, **caractérisé en ce que** le prolongement sacrificiel (66) saillant librement à une arête de masque est limité sous forme incurvée, en particulier en forme d'arc de cercle, dans une région de bord située à l'opposé de l'extrémité de fente proximale (60) de la fente de gravure de canal (38).

8. Procédé selon la revendication 6 ou 7, **caractérisé en ce que** les dimensions du prolongement sacrificiel (66) sont adaptées à la largeur de sous-gravure de l'agent de gravure, de telle manière que le prolongement sacrificiel (66) soit gravé entièrement jusqu'à sa base proximale.

9. Procédé selon l'une quelconque des revendications 1 à 8, **caractérisé en ce que** le masque de gravure (10) présente sur sa face opposée (46) située à l'opposé de la fente de gravure de canal (38) une ouverture auxiliaire (50), en particulier pour éviter de créer des arêtes en contre-dépouille dans la région de la pointe (18).

10. Procédé selon la revendication 9, **caractérisé en ce que** l'ouverture auxiliaire (50) est disposée dans une région de formation de pointe (32') du masque de gravure (10) à distance latérale d'une arête de masque.

11. Procédé selon la revendication 9 ou 10, **caractérisé en ce que** l'ouverture auxiliaire (50) présente deux bras d'ouverture auxiliaire (50') s'étendant l'un vers l'autre en forme de V en direction distale.

12. Procédé selon l'une quelconque des revendications 9 à 11, **caractérisé en ce que** l'ouverture auxiliaire (50) présente un prolongement distal d'ouverture auxiliaire (50") s'étendant en direction distale, réalisé de préférence en forme de fente ou de rangée de trous.

13. Procédé selon la revendication 12, **caractérisé en ce que** l'extrémité proximale du prolongement d'ouverture auxiliaire (50") est disposée, vue en direction proximale, derrière l'extrémité distale (44) de la pointe à former (18).

14. Procédé selon l'une quelconque des revendications 9 à 13, **caractérisé en ce que** l'ouverture auxiliaire (50) est réalisée en forme de Y, dans lequel le point de jonction (56) des branches d'ouverture s'étendant l'une vers l'autre est disposé à une distance distale du contour de pointe à former (52) de la face opposée du canal (46).

15. Organe de piqûre (14) avec une tige allongée (16), une pointe d'extrémité distale (18), et un canal de collecte (22) ouvert latéralement pour un liquide corporel, qui s'étend le long de la tige (16) jusque dans la région de la pointe (16), dans lequel le canal de collecte (22) s'étend à au moins une partie d'extrémité (40, 60) avec une section transversale décroissant de façon continue, **caractérisé en ce qu'**une partie de maintien proximale (20) est formée avec une arête de bride (68) formant le lieu d'embouchure du canal de collecte (22) par un procédé de gravure par masque selon l'une quelconque des revendications précédentes.
